# EUROPEAN PATENT APPLICATION

(11) **EP 1 712 555 A1**
(43) Date of publication of application: **18.10.2006**
(21) Application number: 05709613.3
(22) Date of filing: 02.02.2005
(51) Int. Cl.: C07H 15/203, A61K 31/7036, A61P 1/04

(54) **PRODRUG COMPRISING 5-AMINOSALICYLATE GLYCOSIDE**

(30) Priority: 03.02.2004 JP 2004026916
(71) Applicant: Nippon Shinyaku Co., Ltd., Kyoto-shi, Kyoto 601-8550 (JP)
(72) Inventor: NAKAMURA, Akio, Minami-ku, Kyoto-shi, Kyoto 6018465 (JP)
(74) Representative: Vossius & Partner
(86) International application number: PCT/JP2005/001492
(87) International publication number: WO 2005/075492

(57) **Abstract**

An object of the present invention is to provide a therapeutic agent for ulcerative colitis which allows 5-aminosalicylic acid (5-ASA) useful as a therapeutic agent for ulcerative colitis to be efficiently delivered to the large intestine of the affected site almost without being absorbed or metabolized in the stomach or the upper part of the small intestine and is safe and can be administered over a long term. The present invention relates to 5-ASA into which D-galactose represented by the following general formula [1] has been introduced.

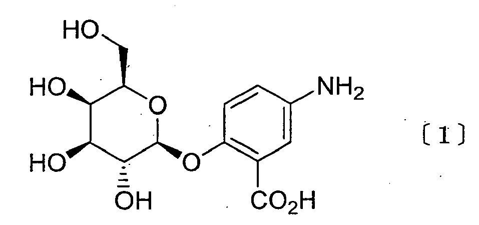

The compound of the present invention can be efficiently delivered to the large intestine of the site of action, and is degraded by the intestinal bacterial flora, whereby 5-ASA as the active ingredient can be produced in the large intestine.

## Description

### Technical Field

The present invention relates to 5-amino-2-(β-D-galactopyranosyloxy)benzoic acid (hereinafter referred to as "compound [1]") represented by the following general formula [1] or 5-amino-2-(α-D-galactopyranosyloxy)benzoic acid (hereinafter referred to as "compound [2]") represented by the following general formula [2] or a pharmaceutically acceptable salt thereof.

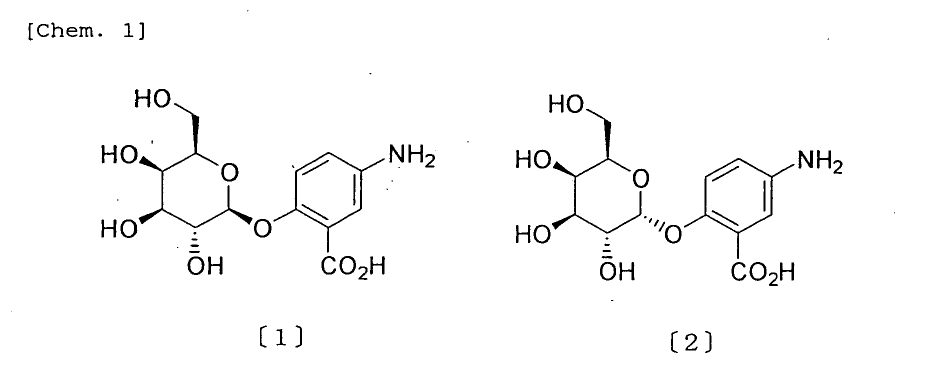

Moreover, the present invention relates to a pharmaceutical composition comprising as an active ingredient the compound [1] or the compound [2] or a pharmaceutically acceptable salt thereof.

Further, the present invention relates to a therapeutic agent for ulcerative colitis comprising as an active ingredient the compound [1], the compound [2] or 5-amino-2-(β-D-glucopyranosyloxy)benzoic acid (hereinafter referred to as "compound [3]") represented by the following general formula [3] or a pharmaceutically acceptable salt thereof.

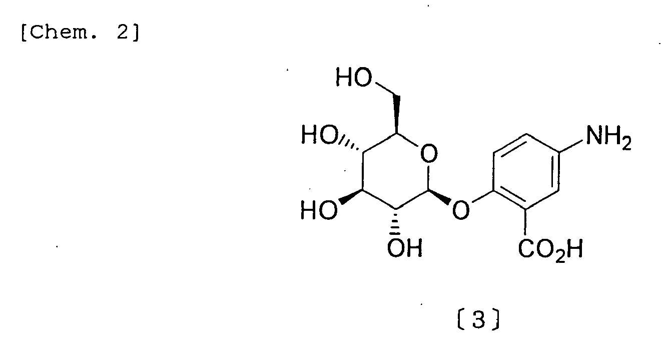

### Background Art

5-Aminosalicylic acid (hereinafter referred to as "5-ASA") has a free radical (DPPHL) reducing action, a hydrogen peroxide scavenging action, a hypochlorite ion scavenging action, inhibitory action on lipid peroxidation and leukotriene B₄ biosynthesis, therefore, it is useful as a therapeutic agent for ulcerative colitis (UC) and Crohn's disease (CD) generically called inflammatory bowel diseases (IBD), which are refractory inflammatory diseases in nature that require lifelong treatment while alternating between remission and exacerbation (see, for example, Non-patent document 1).

However, it is known that orally administered 5-ASA per ser is rapidly and completely absorbed in the upper part of the small intestine, and little amount of 5-ASA, which exhibits its effect by the local action on the inflammatory site, is delivered near to the large intestine of the affected site (see, for example, Non-patent document 3).

In view of this, in order to deliver 5-ASA to the large intestine of the site of action, drug delivery system (hereinafter referred to as "DDS") of 5-ASA and prodrugs of 5-ASA have been studied (see, for example, Non-patent documents 1, 2 and 4).

There exists a DDS preparation of 5-ASA, trade name: Pentasa (registered trademark), manufactured by Nisshin Kyorin Pharmaceutical Co., Ltd., which is so formulated as to gradually release 5-ASA in the area from the small intestine to the large intestine by coating 5-ASA with a porous film of ethyl cellulose (see, for example, Non-patent documents 1 and 2). However, it is known that a considerable amount of 5-ASA is transferred to the plasma after a single oral administration of Pentasa to healthy adult at a dose of 1000 mg as 5-ASA in the fasting state, although the plasma concentration of the unchanged drug is diminished to one-fourteenth level at the lowest (Cmax = 1448.6 ± 586.4 ng/ml) as compared to the case where a single oral administration of 5-ASA per se is given (see, for example, Non-patent document 5).

Further, as a prodrug of 5-ASA, there is Salazosulfapyridine (hereinafter referred to as "SASP") (trade name: Salazopyrin (registered trademark), manufactured by Pfizer Inc.), in which an amino group of 5-ASA is azotized (see, for example, Non-patent document 3). The compound is metabolized to 5-ASA by the intestinal bacteria which exist in the large intestine and have an azoreduction enzyme. Although the effectiveness of SASP for ulcerative colitis has been established, there is a problem that side effects such as drug hypersensitivity, male infertility, nausea and headache are caused by sulfapyridine (SP) which is formed after the degradation of SASP by the intestinal bacteria (see, for example, Non-patent document 3).

Further, as other prodrugs, methyl 5-amino-2-(β-D-glucopyranosyloxy)benzoate and methyl 2-acetoxy-5-(β-D-glucopyranosylamino)benzoate are known which are glucose glycosides of methyl 5-aminosalicylate having high water solubility (see, for example, Non-patent documents 6 and 7). Although the safety of the compounds has been established, their therapeutic effects on ulcerative colitis have not been investigated at all.

Further, apart from 5-ASA, as a prodrug of a steroid compound useful as a therapeutic agent for ulcerative colitis, a glycoside of dexamethasone or prednisolone with glucose or the like has been reported (see, for example, Patent document 1). An object of the said compound is to give a specific drug delivery to the large intestine. However, it has been reported that after intragastrical administration to rats, only 60% of a glucose derivative of dexamethasone is delivered to the cecum and only 15% or less of a glucose derivative of prednisolone is delivered to the cecum.

As described above, at the present, no therapeutic agents for ulcerative colitis are known that are safe, can be administered over a long term, and are able to efficiently deliver 5-ASA useful as a therapeutic agent for ulcerative colitis to the large intestine of the affected site almost without being absorbed or metabolized in the stomach or the upper part of the small intestine.
[Patent document 1] JP-B-60-501105
[Non-patent document 1] Folia Pharmacol. Jpn. 104, pp. 447-457 (1994)
[Non-patent document 2] Folia Pharmacol. Jpn. 104, pp. 303-311 (1994)
[Non-patent document 3] Scandinavian Journal of Gastroenterology, 23, pp. 107-112 (1988)
[Non-patent document 4] Advanced Drug Delivery Reviews, 7, pp. 149-199 (1991)
[Non-patent document 5] Yakuri To Chiryo, 22 (Suppl. 10), pp. S2467-S2495 (1994)
[Non-patent document 6] Magyar Kemiai Folyoirat, 97 (4), pp. 143-148 (1991)
[Non-patent document 7] Archiv der Pharmazie An International Journal Pharmaceutical and Medicinal Chemistry, 332 (9), pp. 321-326 (1999)

### Disclosure of the invention

### Problems that the Invention is to Solve

An object of the present invention is to provide a therapeutic agent for ulcerative colitis which allows 5-ASA useful as a therapeutic agent for ulcerative colitis to be efficiently delivered to the large intestine of the affected site almost without being absorbed or metabolized in the stomach or the upper part of the small intestine and be safely administered over a long term.

### Means for Solving the Problems

As a result of extensive studies, the present inventors have found a compound with which the above-mentioned object can be achieved, thus having completed the present invention.

The present invention may include a compound [1] or a compound [2] or a pharmaceutically acceptable salt thereof.

Further, the present invention may include a pharmaceutical composition comprising a compound [1] or a compound [2] or a pharmaceutically acceptable salt thereof as an active ingredient, and further, a therapeutic agent for ulcerative colitis comprising a compound [1], a compound [2] or a compound [3] (for the sake of convenience, hereinafter collectively referred to as the "compound of the present invention") or a pharmaceutically acceptable salt thereof as an active ingredient.

Because the compound of the present invention is metabolized to 5-ASA by the intestinal bacterial flora in the large intestine, systemic side effects can be reduced and a relatively long-term administration at a relatively high dose has become possible by using the compound of the present invention.

The definition of the term used in the present description is as follows.

"Ulcerative colitis" is an erosive nonspecific inflammation of the large intestine of unknown cause, which mainly attacks the mucosa and often forms erosions and ulcers.

Hereinafter, the present invention will be described in detail.

The compound of the present invention can be produced in accordance with, for example, the following method from a known compound or an intermediate that can be easily prepared. In the production of the compound of the present invention, when a starting material has a substituent affecting a reaction, it is general that the reaction is carried out after the starting material is protected with an appropriate protecting group by a known method in advance. The protecting group can be removed by a known method after the reaction.

### Production method 1

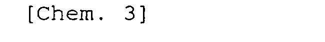

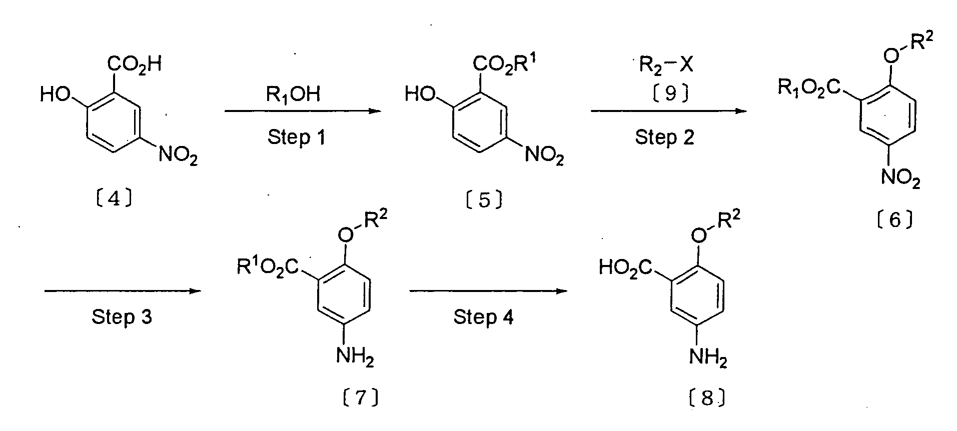

(In the formula, R¹ represents straight-chain or branched-chain alkyl having 1 to 6 carbon atoms; R² represents D-glucopyranosyl or D-galactopyranosyl (each hydroxyl group of R² may be protected with a protecting group such as acetyl); and X represents a halogen atom such as fluorine, chlorine, bromine and iodine.)

### Step 1

This step is esterification of a known compound [4] and can be carried out by a known method (see Non-patent document 7). The reaction temperature is suitably in the range of 20°C to 200°C. In general, a reaction solvent varies depending on the type of carboxylic ester to be produced, but examples thereof may include alcohols such as methanol and ethanol. Examples of an acid may include inorganic acids such as hydrochloric acid and sulfuric acid. The reaction time varies depending on the type of starting material to be used or the reaction temperature, but it is suitably in the range of 1 hour to 72 hours in general.

### Step 2

This step is condensation of a compound [5] with a compound obtained by halogenating glucose or galactose at the anomeric position, and can be carried out by a method known per se (see Non-patent document 7). This reaction proceeds by inversion of the configuration in the presence of a catalyst. Examples of the catalyst for this reaction may include silver oxide (I), mercury oxide (II) and AgOCOR³ (R³ represents straight-chain or branched-chain alkyl having 1 to 6 carbon atoms). In general, a reaction solvent is not particularly limited as long as it is not involved in the reaction, and examples thereof may include quinoline. The reaction temperature is suitably in the range of 0°C to 100°C. The reaction time varies depending on the type of starting material to be used, the reaction temperature and so on, but it is suitably in the range of 1 hour to 72 hours in general. Further, if desired, a protecting group for each hydroxyl group of R² of a produced compound [6] may be removed by a known method.

Further, although the compound [9], which is a starting compound, is commercially available, it can also be produced by, for example, the following method. This reaction is a halogenation reaction at the anomeric position of a sugar such as glucose or galactose, and can be carried out by a method known per se. Examples of halogenating agent may include generally a solution of hydrogen bromide in acetic acid, phosphorus oxybromide, phosphorus oxychloride and the like. In general, a reaction solvent is not particularly limited as long as it is not involved in the reaction, and examples thereof may include halogenated solvents such as methylene chloride, chloroform and 1,2-dichloroethane. The reaction temperature is suitably in the range of 0°C to 100°C. The reaction time varies depending on the type of starting material to be used or the reaction temperature, but it is suitably in the range of 1 hour to 72 hours in general.

### Step 3

This step is hydrogenation of the compound [6], and can be carried out by a method known per se (see Non-patent document 7). This reaction can be carried out, for example, in the presence of a metal catalyst in a suitable solvent, generally under a hydrogen atmosphere of 1 to 10 atm. and at 0°C to 100°C. Examples of the metal catalyst may include generally palladium carbon, palladium black, platinum dioxide, platinum carbon and the like. A reaction solvent is not particularly limited as long as it is not involved in the reaction, and examples thereof may include ethers such as tetrahydrofuran, 1,4-dioxane and 1,2-dimethoxyethane, alcohols such as methanol and ethanol, amides such as N,N-dimethylformamide and N,N-dimethylacetamide, hydrocarbons such as benzene, toluene and xylene, and solvent mixtures thereof. The reaction time varies depending on the type of starting material to be used or the reaction temperature, but it is suitably in the range of 1 hour to 48 hours in general.

Further, if desired, a protecting group for each hydroxyl group of R² of a produced compound [7] may be removed by a known method.

### Step 4

This step is hydrolysis of a carboxylic ester of the compound [7], and can be carried out by a method known per se. The reaction temperature is suitably in the range of 0°C to 100°C. A reaction solvent is not particularly limited as long as it is not involved in the reaction, and examples thereof may include alcohols such as methanol and ethanol. Examples of a base may include inorganic bases such as sodium hydroxide and potassium hydroxide. The reaction time varies depending on the type of starting material to be used or the reaction temperature, but it is suitably in the range of 1 hour to 72 hours in general.

Further, as another method of producing the compound [6], the following method can be exemplified.

### Production method 2

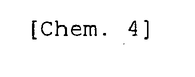

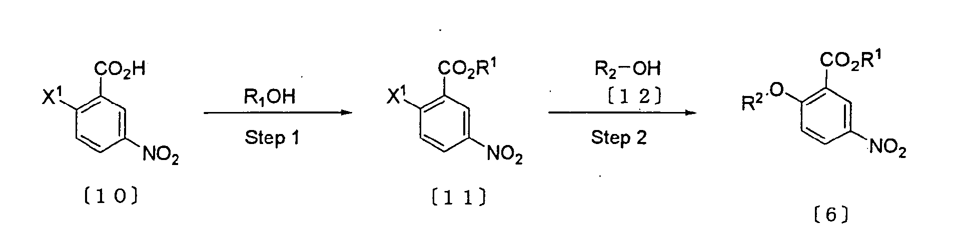

(In the formula, R¹ and R² have the same meaning as described above. X¹ represents a halogen atom such as fluorine, chlorine, bromine or iodine.)

### Step 1

This step is esterification of a known compound [10] and can be carried out by a known method (see Non-patent document 7). The reaction temperature is suitably in the range of 20°C to 200°C. In general, a reaction solvent varies depending on the type of carboxylic ester to be produced, and examples thereof may include alcohols such as methanol and ethanol. Examples of an acid may include inorganic acids such as hydrochloric acid and sulfuric acid. The reaction time varies depending on the type of starting material to be used or the reaction temperature, but it is suitably in the range of 1 hour to 72 hours in general.

### Step 2

This step is condensation of a compound [11] with a glucose or galactose derivative [12], and can be carried out by a method known per se. Examples of a condensing agent for this reaction may include bases. In this reaction, because the configuration of the anomeric position cannot be controlled, it is necessary to separate and purify a single diastereomer by using silica gel chromatography or the like. By this separation procedure, both compounds (α-form and β-form) can be obtained for the configuration of the anomeric position. Examples of the base to be used in this reaction may include 1,5-diazabicyclo[4.3.0]-5-nonene, 1,4-diazabicyclo[2.2.2]octane and 1,8-diazabicyclo[5.4.0]-7-undecene. In general, a reaction solvent is not particularly limited as long as it is not involved in the reaction, and examples thereof may include acetonitrile and dimethylsulfoxide. The reaction temperature is suitably in the range of 0°C to 100°C. The reaction time varies depending on the type of starting material to be used, the reaction temperature and so on, but it is suitably in the range of 1 hour to 72 hours in general. Further, if desired, a protecting group for each hydroxyl group of R² of a produced compound [6] may be removed by a known method.

The compound of the present invention produced in this way can be separated and purified by a method known per se such as concentration, solvent conversion, phase transfer, solvent extraction, crystallization, recrystallization, fractional distillation or chromatography.

The compound of the present invention can be used as a medicament as such, and also it can be converted into a pharmaceutically acceptable salt by a known method.

The "salt" of the compound of the present invention may include a pharmaceutically acceptable salt, for example, salts of inorganic acids such as hydrochloric acid, sulfuric acid, nitric acid, phosphoric acid, hydrofluoric acid and hydrobromic acid, salts of organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid and camphorsulfonic acid, salts of alkali or alkaline earth metals such as sodium, potassium and calcium. Hydrochloride salt is preferable.

A hydrochloride salt of the compound of the present invention can be obtained by treating the compound of the present invention with an alcohol solution of hydrogen chloride or a diethyl ether solution thereof and then obtaining a deposited crystal by filtration, or when a crystal is not deposited, concentrating the solution thereby to deposit a crystal and obtaining the crystal by filtration, for example.

As shown in the test examples described below, the compound of the present invention has an excellent characteristic feature that it hardly is transferred to the plasma, which is not possessed by currently available 5-ASA-related pharmaceutical products, and allows 5-ASA, which is an active ingredient, to be efficiently delivered widely to the affected sites, i.e. the cecum and throughout the large intestine over the proximal colon, the distal colon and the rectum after oral administration. Therefore, the compound of the present invention is useful as a therapeutic agent for ulcerative colitis which is safe and can be administered over a long term. In particular, the compound [1] has a prominent effect.

In a trinitrobenzenesulfonic acid (TNBS) -induced colitis model rat as shown in the test examples described below, the compound [1] of the present invention significantly suppresses the damage score and the wet weight of the large intestine and is shown to be an excellent therapeutic agent for ulcerative colitis.

In the case that the compound of the present invention is administrated as a medicine, the compound of the present invention or a pharmaceutically acceptable salt thereof can be administrated to mammals including humans as it is or as a pharmaceutical composition containing the compound in a pharmaceutically acceptable nontoxic and inactive carrier at an amount of, for example, 0.1% to 99.5%, preferably 0.5% to 90%.

The pharmaceutically acceptable carrier may include diluents and fillers in the form of solid, semisolid, or liquid, and other formulation auxiliaries, and at least one of them is employed. The pharmaceutical composition is preferably administrated in a dosage form. The pharmaceutical composition can be administrated orally or parenterally (e.g., transrectal administration, etc.). It is, of course, administrated in the suitable dosage form for these administration methods. For example, oral administration is preferred.

The dosage of the compound of the present invention may be preferably adjusted in view of conditions of a patient such as age, body weight, character and severity of disease, as well as the route of administration, but is usually in the range of 10 mg/adult to 10 g/adult, preferably 1 g to 4 g/adult per day in the case of oral administration as an effective amount of the compound of the present invention or a pharmaceutically acceptable salt thereof. In some cases, a dosage less than the above range may be sufficient or a dosage more than the above range may be required. Usually, the daily dosage may be administrated once a day or several times as being divided into portions.

The oral administration can be carried out in a solid or liquid unit dosage form, such as a particle, a powder, a tablet, a sugar-coated tablet, a capsule, a granule, a suspension, a liquid, a syrup, a drop, a sublingual tablet, or other dosage forms.

The particle can be produced by pulverizing the compound of the present invention or a pharmaceutically acceptable salt thereof into a suitable particle size.

The powder can be produced by pulverizing the compound of the present invention or a pharmaceutically acceptable salt thereof into a suitable particle size and subsequently mixing it with a pharmaceutical carrier, such as an edible carbonhydrate such as starch or mannitol and others. Those which may be mixed, if necessary, are flavors, preservatives, dispersants, colorants, fragrances or the like.

The capsule can be produced by filling an envelope of capsule such as gelatin capsule with the particle or powder which have been pulverized as above or granulated one to be described in the section of the tablet. A lubricant or a fluidizer, e.g., colloidal silica, talc, magnesium stearate, calcium stearate, solid polyethylene glycol may be added to the powdery one and then the filling operation may be conducted. When a disintegrator or a solubilizer, e.g., carboxymethylcellulose, carboxymethylcellulose calcium, hydroxypropylcellulose having a low degree of substitution, crosscarmellose sodium, carboxymethylstarch sodium, calcium carbonate, or sodium carbonate is added, effectiveness of the medicine when the capsule is taken can be improved.

The tablet can be produced by preparing a powdery mixture together with an excipient, granulating or slugging it, adding a disintegrator or a lubricant thereto, and then compacting into a tablet. The powdery mixture can be produced by mixing an appropriately pulverized material with the above diluent or a base and, if necessary, a binder (e.g., carboxymethylcellulose sodium, methylcellulose, hydroxypropylmethylcellulose, gelatin, polyvinylpyrrolidone, or polyvinyl alcohol), a dissolution retardant (e.g., paraffin), a re-absorbent (e.g., a quaternary salt), or an adsorbent (e.g., bentonite, kaolin, dicalcium phosphate) can be used in combination. The powdery mixture can be first wetted with a binder such as syrup, starch glue, gum arabic, a cellulose solution, and a polymer solution, followed by mixing with stirring, and then drying and grinding. Instead of the procedure for granulating the powder as described above, after it is subjected to a tablet compacting machine, the resulting slugs in an incomplete form can be crashed to form granules. The granules thus formed can be prevented from one another's attachment by adding stearic acid, a stearate salt, talc, mineral oil, or the other substance as a lubricant. The mixture thus lubricated is then tableted. The uncoated tablets thus produced can be subjected to film coating or sugar coating.

Moreover, the compound of the present invention or a pharmaceutically acceptable salt thereof may be directly compacted directly into tablets after mixing with a fluid inactive carrier without steps of granulation and slug formation as described above. A transparent or translucent protective film in the form of a shellac sealing film, a film of a sugar or polymer material, a glossy film composed of wax may also be used. The other orally administration forms, such as a solution, a syrup and an elixir may be formulated as a unit dosage form so that a certain amount thereof may contain a certain amount of a medicament. The syrup can be produced by dissolving the compound of the present invention or a pharmaceutically acceptable salt thereof in an appropriate flavored aqueous solution, while the elixir can be produced by using a nontoxic alcoholic carrier.

If necessary, a dosage unit formulation for oral administration may be microencapsulated. An elongated working time and sustained release of the active ingredient may also be achieved by coating the formulation or embedding it in a polymer, a wax, or the like.

In the parenteral administration, a suppository, or the like may be used. The transrectal administration can be achieved using a suppository produced by dissolving or suspending the compound of the present invention or a pharmaceutically acceptable salt thereof in a low-melting water-soluble or water-insoluble solid, such as polyethylene glycol, cacao butter, a semi-synthesized oil and fat (e.g., Witepsol/registered trademark), a higher ester (e.g., myristyl palmitate), or a mixture thereof.

### Brief Description of the Drawings

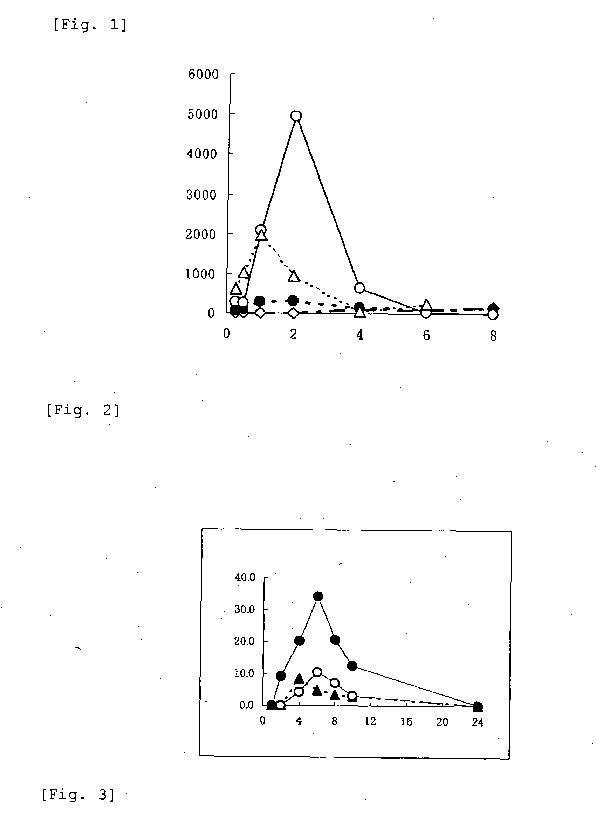

[Fig. 1] Fig. 1 shows the change of plasma concentration of 5-ASA. The vertical axis represents the rat plasma concentration of 5-ASA (ng/ml) and the horizontal axis represents time (hour). The black circles, the white lozenges, the white triangles, and the white circles represent the change of concentration of 5-ASA after the administration of the compound [1], the compound [2], the compound [3], and Pentasa (registered trademark), respectively.
[Fig. 2] Fig. 2 shows the change of the amount of 5-ASA in the cecal contents. The vertical axis represents the amount of 5-ASA (% of dose) present in the rat cecal contents and the horizontal axis represents time (hour). The black circles, the white circles, and the black triangles represent the change of the amount of 5-ASA after the administration of the compound [1], Pentasa (registered trademark) and 5-ASA, respectively.

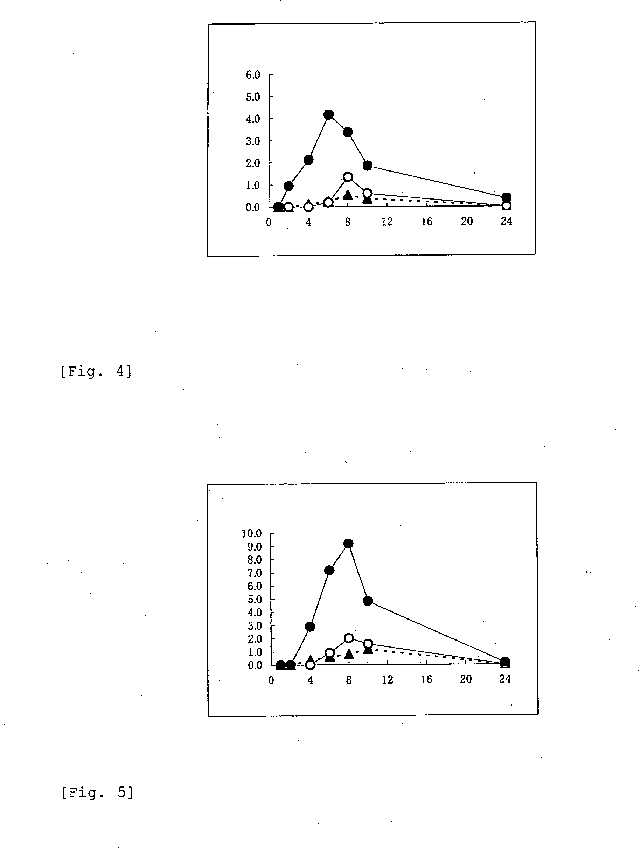

[Fig. 3] Fig. 3 shows the change of the amount of 5-ASA in the proximal colonic contents. The vertical axis represents the amount of 5-ASA (% of dose) present in the rat proximal colonic contents and the horizontal axis represents time (hour). The black circles, the white circles, and the black triangles represent the change of the amount of 5-ASA after the administration of the compound [1], Pentasa (registered trademark) and 5-ASA, respectively.
[Fig. 4] Fig. 4 shows the change of the amount of 5-ASA in the distal colonic contents. The vertical axis represents the amount of 5-ASA (% of dose) present in the rat distal colonic contents and the horizontal axis represents time (hour). The black circles, the white circles, and the black triangles represent the change of the amount of 5-ASA after the administration of the compound [1], Pentasa (registered trademark) and 5-ASA, respectively.
[Fig. 5] Fig. 5 shows the change of the amount of 5-ASA in the rectal contents. The vertical axis represents the amount of 5-ASA (% of dose) present in the rat rectal contents and the horizontal axis represents time (hour). The black circles, the white circles, and the black triangles represent the change of the amount of 5-ASA after the administration of the compound [1], Pentasa (registered trademark) and 5-ASA, respectively.

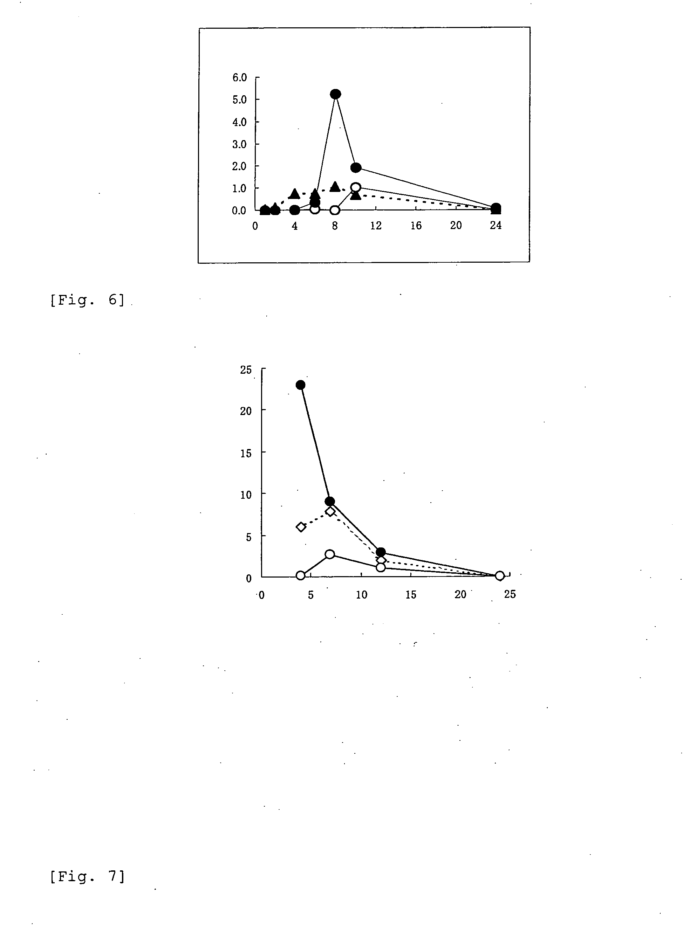

[Fig. 6] Fig. 6 shows the change of the concentration of 5-ASA in the colonic tissue. The vertical axis represents the concentration of 5-ASA (µg/g) present in 1 g of rat colon and the horizontal axis represents time (hour). The white lozenges, and the white circles represent the change of the concentration of 5-ASA represent the change of the concentration of 5-ASA after the administration of the compound [2], and Pentasa (registered trademark), respectively.

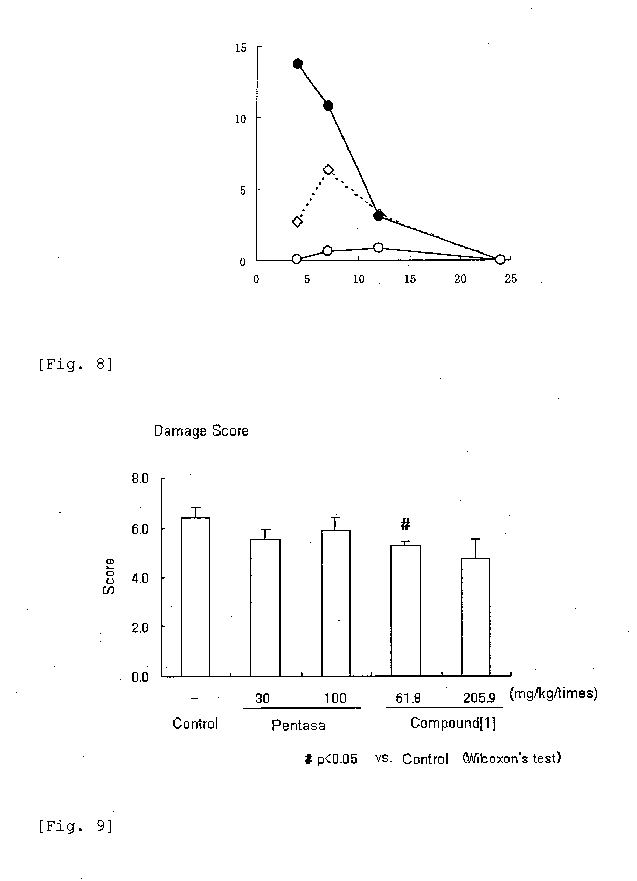

[Fig. 7] Fig. 7 shows the change of the concentration of 5-ASA in the rectal tissue. The vertical axis represents the concentration of 5-ASA (µg/g) present in 1 g of the rat rectum and the horizontal axis represents time (hour). The white lozenges, and the white circles represent the change of the concentration of 5-ASA after the administration of the compound [2], and Pentasa (registered trademark), respectively.
[Fig. 8] Fig. 8 shows the therapeutic effects of Pentasa (registered trademark) and the compound [1] on TNBS-induced colitis in rats by the damage score. The vertical axis represents the damage score and the horizontal axis represents the dose of each test drug (mg/kg/once).

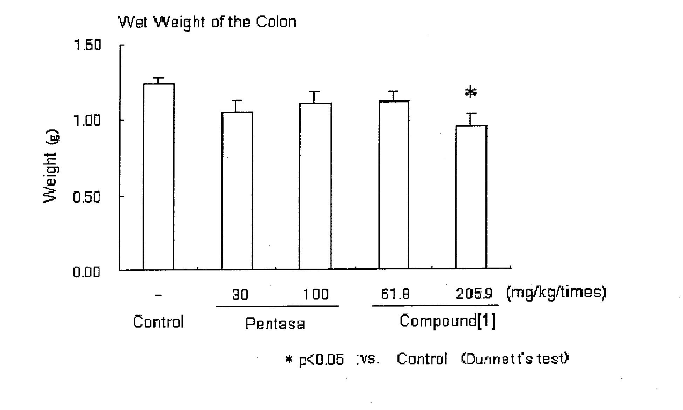

[Fig. 9] Fig. 9 shows the effects of Pentasa (registered trademark) and the compound [1] on TNBS-induced colitis in rats against an increase in the wet weight of tissue accompanying the colitis onset. The vertical axis represents the wet weight of large intestine (g) and the horizontal axis represents the dose of each test drug (mg/kg/once).

### Best Mode for Carrying Out the Invention

The present invention will be illustrated further in detail below with reference to Examples, Test examples and Formulation examples, but the present invention is not limited thereto.

### Example 1: 5-Amino-2-(β-D-galactopyranosyloxy)benzoic acid

### Step 1: Methyl 5-nitrosalicylate

To a solution of 30 g of 5-nitrosalicylic acid in 500 ml of anhydrous methanol, concentrated sulfuric acid was added dropwise, and the mixture was heated to reflux for 2 days. The reaction solution was concentrated under reduced pressure and diluted with 500 ml of ethyl acetate, and 500 ml of water was added thereto. Then, a saturated sodium bicarbonate solution was slowly added thereto under cooling with ice to make the solution alkaline (pH = 9). The deposited yellow precipitate was filtered, and the aqueous layer of the filtrate was subjected to an extraction with ethyl acetate. The combined organic layer was washed with water and saturated brine and dried over anhydrous magnesium sulfate and then filtered. Then, the solvent was concentrated, whereby 31.26 g of methyl 5-nitrosalicylate was obtained.

### Step 2-1: 2',3',4',6'-Tetra-O-acetyl-α-D-galactopyranosyl bromide

A solution of 65 g of 1',2',3',4',6'-penta-O-acetyl-β-D-galactopyranose in 500 ml of methylene chloride was cooled with ice, and 177.5 g of 30% hydrogen bromide/acetic acid solution was added dropwise thereto. The reaction mixture was stirred at room temperature for 14 hours, and then poured into a saturated sodium bicarbonate solution containing ice. The organic layer was washed with saturated brine and dried over anhydrous magnesium sulfate and then filtered. Then, the solvent was concentrated, whereby 68.7 g of 2',3',4',6'-tetra-O-acetyl-α-D-galactopyranosyl bromide was obtained.

### Step 2-2: Methyl 5-nitro-2-(2',3',4',6'-tetra-O-acetyl-β-D-galactopyranosyloxy)benzoate

To a solution of 30.55 g of methyl 5-nitrosalicylate obtained in the step 1 and 63.7 g of 2',3',4',6'-tetra-O-acetyl-α-D-galactopyranosyl bromide obtained in the step 2-1 in 250 ml of quinoline, 35.92 g of silver oxide was added, and the mixture was stirred at room temperature for 62 hours under dark condition. The reaction mixture was diluted with 1000 ml of ethyl acetate, and then, celite filtration was carried out. After the ethyl acetate layer was washed twice with 1000 ml of 2 N hydrochloric acid, the aqueous layer was subjected to an extraction twice with ethyl acetate. The combined organic layer was washed with a saturated sodium bicarbonate solution, water and saturated brine, and dried over sodium sulfate, and then filtered. Then, the solvent was concentrated, whereby 71.7 g of methyl 5-nitro-2-(2',3',4',6'-tetra-O-acetyl-β-D-galactopyranosyloxy)benzoate was obtained.

### Step 2-3: Methyl 5-nitro-2-(β-D-galactopyranosyloxy) benzoate

A methanol solution containing 10.55 g of methyl 5-nitro-2-(2',3',4',6'-tetra-O-acetyl-β-D-galactopyranosyloxy)benzoate obtained in the step 2-2 was stirred at 60°C, and sodium methoxide was added thereto. After the mixture was stirred for 30 minutes, the reaction mixture was neutralized with 5.0 g of Amberlite IRC-50. After filtration was carried out, the organic layer was concentrated, whereby 4.90 g of methyl 5-nitro-2-(β-D-galactopyranosyloxy)benzoate was obtained.

### Step 3: Methyl 5-amino-2-(β-D-galactopyranosyloxy) benzoate

To a solution of 4.90 g of methyl 5-nitro-2-(β-D-galactopyranosyloxy)benzoate obtained in the step 2-3 in 100 ml of methanol, 0.49 g of 10% palladium carbon was added, and a catalytic reduction was carried out at room temperature under a hydrogen atmosphere of 1 atm. After 20 hours, the reaction solution was filtered to remove the catalyst, and the organic layer was concentrated, whereby 4.18 g of methyl 5-amino-2-(β-D-galactopyranosyloxy) benzoate was obtained.

### Step 4: 5-Amino-2-(β-D-galactopyranosyloxy)benzoic acid

To a suspension of 4.18 g of methyl 5-amino-2-(β-D-galactopyranosyloxy)benzoate obtained in the step 2-4 in 120 ml of anhydrous methanol, 12.7 ml of 1 N aqueous sodium hydroxide solution was added dropwise. The mixture was stirred while heating to reflux for 16 hours. The reaction solution was concentrated under reduced pressure as such, and the residue was diluted with distilled water. Then, the solution was neutralized with 6.4 ml of 2 N hydrochloric acid. The mixture was concentrated, whereby 3.41 g of the objective compound was obtained.
Colorless powder
MS (EI): m/z = 338 [M + Na]⁺
Rotation: [α]_{D}²⁰ = -19.84 (C = 1.28, H₂O)

| Elemental analysis values (as C₁₃H₁₇NO₈) | | | |
|---|---|---|---|
| Calculated (%): | C: 49.52, | H: 5.43, | N: 4.44 |
| Found (%): | C: 49.12, | H: 5.37, | N: 4.38 |

¹H NMR (D₂O) :
3.74 to 4.01 (m, 6H, H-2 to 6), 5.04 (d, 1H, J_{1,2} = 7.4 Hz, H-1), 7.30 to 7.39 (m, 3H, Ph)

### Example 2: 5-Amino-2-(α-D-galactopyranosyloxy)benzoic acid

### Step 1: Methyl 2-fluoro-5-nitrobenzoate

A solution containing 12.0 g of 2-fluoro-5-nitrobenzoic acid, 60 ml of anhydrous tetrahydrofuran and 60 µl of dimethylformamide was cooled with ice, and 9.05 g of oxalyl chloride was added dropwise thereto. After completion of the dropwise addition, the mixture was stirred at room temperature for 5 hours. To the reaction solution, 30 ml of anhydrous tetrahydrofuran and 30 ml of a methanol solution were added dropwise, and the mixture was stirred overnight at room temperature. The reaction solution was concentrated under reduced pressure and diluted with 240 ml of ethyl acetate. Then, the diluted solution was washed with 5% aqueous sodium bicarbonate solution and saturated brine and dried over anhydrous magnesium sulfate and then filtered. Then, the solvent was concentrated, and to the concentrated residue, 24 ml of isopropyl ether was added to dissolve the residue. Then, the solution was cooled to 5°C to deposit a crystal. The deposited crystal was filtered under reduced pressure and dried at room temperature under reduced pressure, whereby 10.5 g of methyl 2-fluoro-5-nitrobenzoate was obtained.

### Step 2: Methyl 5-nitro-2-(2',3',4',6'-tetra-0-acetyl-α-D-galactopyranosyloxy)benzoate

To a solution of 7.13 g of methyl 2-fluoro-5-nitrobenzoate obtained in the step 1 and 12.50 g of 2',3',4',6'-tetra-O-acetyl-D-galactopyranose in 70 ml of acetonitrile, 4.95 g of DBU was added dropwise, and the mixture was stirred at room temperature for 2 hours. The reaction solution was concentrated under reduced pressure, diluted with 300 ml of ethyl acetate, washed with 150 ml of 1 N hydrochloric acid, 150 ml of 5% aqueous sodium bicarbonate solution and 150 ml of saturated brine, and dried over anhydrous magnesium sulfate and then filtered. Then, the solvent was concentrated. The concentrate was purified with a column chromatograph (Wako gel (registered trademark) C-200 (manufactured by Wako Pure Chemical Industries, Ltd.), n-hexane : ethyl acetate = 4 to 1.5), whereby 7.51 g of methyl 5-nitro-2-(2',3',4',6'-tetra-O-acetyl-α-D-galactopyranosyloxy)benzoate and 7.92 g of methyl 5-nitro-2-(2',3',4',6'-tetra-O-acetyl-β-D-galactopyranosyloxy)benzoate were obtained.

### Step 3-1: Methyl 5-amino-2-(2',3',4',6'-tetra-O-acetyl-α-D-galactopyranosyloxy)benzoate

To a solution of 7.00 g of methyl 5-nitro-2-(2',3',4',6'-tetra-O-acetyl-α-D-galactopyranosyloxy) benzoate obtained in the step 2-1 in 210 ml of methanol, 0.70 g of 10% palladium carbon was added, and a catalytic reduction was carried out at room temperature under a hydrogen atmosphere of 1 atm. After 18 hours, the reaction solution was filtered to remove the catalyst, and the organic layer was concentrated. The concentrate was purified with a column chromatograph (Wako gel (registered trademark) C-200 (manufactured by Wako Pure Chemical Industries, Ltd.), n-hexane : ethyl acetate = 3:1 to 3:2), whereby 5.73 g of methyl 5-amino-2-(2',3',4',6'-tetra-O-acetyl-α-D-galactopyranosyloxy)benzoate was obtained.

### Step 3-2: Methyl 5-amino-2-(α-D-galactopyranosyloxy) benzoate

To 5.52 g of methyl 5-amino-2-(2',3',4',6'-tetra-O-acetyl-α-D-galactopyranosyloxy)benzoate obtained in the step 3-1 in 110 ml of anhydrous tetrahydrofuran and anhydrous methanol (1:1), 307 mg of potassium carbonate was added, and the mixture was stirred at room temperature for 15 hours. The reaction solution was concentrated under reduced pressure, and the concentrate was purified with a column chromatograph (Wako gel (registered trademark) C-200 (manufactured by Wako Pure Chemical Industries, Ltd.), chloroform : methanol = 10:1 to 5:1), whereby 2.71 g of methyl 5-amino-2-(α-D-galactopyranosyloxy)benzoate was obtained.

### Step 9: 5-Amino-2-(α-D-galactopyranosyloxy)benzoic acid

A suspension of 2.00 g of methyl 5-amino-2-(α-D-galactopyranosyloxy)benzoate obtained in the step 3-2 in 40 ml of water, 6.07 ml of 1 N aqueous sodium hydroxide solution was added dropwise, and the mixture was stirred at 50°C for 2 hours. The reaction solution was filtered to remove insoluble substances, and the filtrate was neutralized by adding 6.07 ml of 1 N hydrochloric acid thereto. The reaction solution was concentrated under reduced pressure, whereby 1.34 g of the objective compound was obtained.
Light yellow powder
MS (FAB): m/z = 316 [M + 1]⁺
Rotation: [α]_{D}²⁰ = 79.37 (C = 1.28, H₂O)

| Elemental analysis value (as C₁₃H₁₇NO₈·0.8H₂O) | | | |
|---|---|---|---|
| Calculated (%): | C: 47.36, | H: 5.69, | N: 4.25 |
| Found (%): | C: 47.20, | H: 5.48, | N: 4.22 |

¹H NMR (D₂O) :
3.70 to 4.10 (m, 6H, H-2 to 6), 5.76 (d, 1H, J_{1,2} = 3.6 Hz, H-1), 7.37 to 7.40 (m, 3H, Ph)

### Reference example 1: 5-Amino-2-(β-D-glucopyranosyloxy) benzoic acid

### Step 1: Methyl 5-nitrosalicylate

Synthesis was carried out by using the same method as in the step 1 of Example 1.

### Step 2: Methyl 5-nitro-2-(2',3',4',6'-tetra-O-acetyl-β-D-glucopyranosyloxy)benzoate

To a solution of 6.0 g of methyl 5-nitrosalicylate obtained in the step 1 and 18.8 g of 2',3',4',6'-tetra-O-acetyl-α-D-glucopyranosyl bromide in 60 ml of quinoline, 10.5 g of silver oxide was added, and the mixture was stirred vigorously at room temperature for 1 hour. The reaction mixture was diluted with 300 ml of ethyl acetate, and then, celite filtration was carried out. After the ethyl acetate layer was washed twice with 2 ml of 2 N hydrochloric acid, the aqueous layer was subjected to an extraction twice with 300 ml of ethyl acetate. The combined organic layer was washed with a saturated sodium bicarbonate solution, water and saturated brine, and dried over sodium sulfate, and then filtered. Then, the solvent was concentrated, whereby 15.63 g of methyl 5-nitro-2-(2',3',4',6'-tetra-O-acetyl-β-D-glucopyranosyloxy) benzoate was obtained.

### Step 3-1: Methyl 5-amino-2-(2',3',4',6'-tetra-O-acetyl-β-D-glucopyranosyloxy)benzoate

To a suspension of 12.0 g of methyl 5-nitro-2-(2',3',4',6'-tetra-O-acetyl-β-D-glucopyranosyloxy) benzoate obtained in the step 2 in 400 ml of methanol, 2.4 g of 10% palladium carbon was added, and a catalytic reduction was carried out at 30°C under a hydrogen atmosphere of 3 atm. After 3 hours, celite filtration of the reaction solution was carried out. The solvent was concentrated, whereby 11.2 g of methyl 5-amino-2-(2' ,3' ,4' ,6'-tetra-O-acetyl-β-D-glucopyranosyloxy) benzoate was obtained.

### Step 3-2: Methyl 5-amino-2-(β-D-glucopyranosyloxy) benzoate

To 16 ml of a solution of anhydrous tetrahydrofuran and methanol (1:1) containing 0.68 g of methyl 5-amino-2-(2' ,3' ,4' ,6'-tetra-O-acetyl-β-D-glucopyranosyloxy) benzoate obtained in the step 3-1, 37.8 mg of potassium carbonate was added, and the mixture was stirred overnight at room temperature. To the reaction solution, 0.14 ml of 4 N hydrogen chloride/ethyl acetate solution was added dropwise, and the solvent was concentrated as such. The resulting crude product was purified by silica gel column chromatography (Wako gel (registered trademark) C-200 (manufactured by Wako Pure Chemical Industries, Ltd.), methylene chloride : methanol = 10:1 to 8:1 to 5:1), whereby 332 mg of methyl 5-amino-2-(β-D-glucopyranosyloxy) benzoate was obtained.

### Step 4: 5-Amino-2-(β-D-glucopyranosyloxy)benzoic acid

To a suspension of 100 mg of methyl 5-amino-2-(β-D-glucopyranosyloxy)benzoate obtained in the step 3-2 in 3 ml of methanol, 0.3 ml of 1 N aqueous sodium hydroxide solution was added dropwise, and the mixture was stirred at 50°C. After 5 hours, the temperature was returned to room temperature, and the solvent was distilled off under reduced pressure. The resulting oily residue was dissolved in 1 ml of water, and 0.3 ml of 1 N hydrochloride acid was added dropwise thereto while stirring and cooling with ice. This solution was concentrated to about 1/3 of the initial volume, and the deposited matter was obtained by filtration, whereby 93 mg of the objective compound was obtained.

| Elemental analysis values (as C₁₃H₁₇NO₈·0.2H₂O) | | | |
|---|---|---|---|
| Calculated (%): | C: 48.97, | H: 5.50, | N: 4.39 |
| Found (%): | c: 48.80, | H: 5.35, | N: 4.31 |

### Test example 1: Measurement of plasma concentration of 5-ASA

To SD male rats at 7 weeks of age, 5-ASA was intravenously administered as a test drug, and the compound [1], the compound [2], the compound [3] or Pentasa (registered trademark) was orally administered at a dose of 50 mg/kg as5-ASA. The plasma concentration of 5-ASA was measured by high performance liquid chromatography (HPLC). As for Pentasa (registered trademark), granules obtained by pulverizing Pentasa (registered trademark) tablet were used.

The results are shown in Table 1.

**[Table 1]**

| Pharmacokinetic parameter values based on plasma levels of 5-ASA in rats | | | | | | |
|---|---|---|---|---|---|---|
| Compound | Route | Dose (mg/kg) | Time to maximum plasma concentration (Tmax) (hr) | Maximum plasma concentration (Cmax) (ng/mL) | Area under the curve of concentration (AUC) (ng·hr/mL) | Bioavailability¹⁾ (BA) (%) |
| 5-ASA | i.v. | 1 | - | - | 1417 | - |
| Pentasa | p.o. | 50 | 2 | 4958 | 10441 | 15 |
| Compound[1] | p.o. | 50 | 0.5 | 435 | 1504 | 2 |
| Compound[2] | p.o. | 50 | 8 | 160 | 571 | 0.8 |
| Compound[3] | p.o. | 50 | 1 | 1962 | 4235 | 6 |

| | | | | | | |
|---|---|---|---|---|---|---|
| n = 2 to 3 1) (AUC after oral administration of each test compound / AUC after intravenous administration of 5-ASA) x (intravenously administered amount of 5-ASA / orally administered amount of each test compound) x 100 | | | | | | |

The above result shows that 5-ASA was detected in the plasma at a relatively high concentration after the oral administration of Pentasa (registered trademark)(see Fig. 1), and hence 5-ASA is released from a portion of the administered Pentasa in the upper part of the small intestine as the absorption site.

On the other hand, the plasma concentrations of 5-ASA after the oral administrations of the compound [1], the compound [2] and the compound [3], each of which is a glycoside of 5-ASA, remained low as compared with those of Pentasa (registered trademark) (see Fig. 1). In addition, when the compound (1) or the compound (2) was administered, the plasma concentrations of 5-ASA were remained low as compared with the case where the compound [3] was administered, and little amount of 5-ASA was detected (see Fig. 1). The bioavailability values of 5-ASA (see Table 1) after the oral administration of the compound [1], the compound [2], the compound [3] and Pentasa (registered trademark) were calculated to be 2, 0.8, 6 and 15%, respectively. Namely, the compound [1], the compound [2] and the compound [3] were found to have lower absorption rate from the gastrointestinal tract as compared with Pentasa. In particular, the compound [1] and the compound [2] were found to have remarkably low bioavailability values.

The probable reason for this is that the compound [1] and the compound [2] are not easily hydrolyzed in the stomach and the small intestine as compared with the compound [3] and they do not produce 5-ASA in the stomach and the upper part of the small intestine.

### Test example 2: Change of the concentration of 5-ASA in the gastrointestinal tract contents

To SD male rats at 7 weeks of age, the compound [1], Pentasa (registered trademark) and 5-ASA were orally administered at a dose of 50 mg/kg as 5-ASA, and the contents in the cecum, the proximal colon, the distal colon and the rectum were homogenated and centrifuged. Then, the amount of 5-ASA in the respective sites of the large intestine was measured by high performance liquid chromatography (HPLC). As for Pentasa (registered trademark), granules obtained by pulverizing Pentasa (registered trademark) tablet were used.

The results are shown in Fig. 2 to Fig. 5.

The amount of 5-ASA in the respective sites of the large intestine showed the highest value when the compound [1] was administered (see Figs. 2, 3, 4 and 5).

Further, the compound [2] and Pentasa (registered trademark) were orally administered at a dose of 50 mg/kg as 5-ASA, and the colon and the rectum were homogenated and centrifuged. Then, the concentrations of 5-ASA in the colonic tissue and the rectal tissue were measured by high performance liquid chromatography (HPLC). As for Pentasa (registered trademark), granules obtained by pulverizing Pentasa (registered trademark) tablet were used.

The results are shown in Fig. 6 and Fig. 7.

Also in the case of the compound (2), the concentrations of 5-ASA in the colonic tissue and the rectal tissue were higher as compared with Pentasa (registered trademark) (see Figs. 6 and 7).

In Test example 1, it has been confirmed that the compound [1] and the compound [2] are not easily hydrolyzed in the stomach and the small intestine, do not produce 5-ASA in the stomach and the upper part of the small intestine, and are low in absorption rate from the gastrointestinal tract.

From the results of Test example 2, it was revealed that the compound [1] and the compound [2] are delivered to the large intestine of the affected site and are metabolized to 5-ASA by intestinal bacteria. In particular, as for the compound [1], 5-ASA which is effective for ulcerative colitis was detected at high concentrations in the respective sites of the large intestine.

### Test example 3: Investigation of the effect of the compound [1] on trinitrobenzenesulfonic acid (hereinafter referred to as "TNBS")- induced colitis in rats

To a female SD rat fasted for 24 hours, an aqueous solution of TNBS/50% ethanol (30 mg/0.25 ml/rat) was administered in the colon at 8 cm from the anus using a probe for oral administration under pentobarbital anesthesia to induce colitis. At 3 days after the administration of TNBS, the colon was excised, and the wet weight of the colon of 8 cm long from the anus was weighed. The degree of colitis onset was scored in accordance with the method of (see R.SH Mikhail, S. (Gastroenterology 96, 29-36 (1989)). As the test compounds, Pentasa (registered trademark) at doses of 30 mg/kg and 100 mg/kg and compound [1] at doses of 61.8 mg/kg and 205.9 mg/kg (corresponding to 30 mg/kg and 100 mg/kg in terms of 5-ASA) were orally administered twice a day (on the day of TNBS administration, once at 4 hours before TNBS administration). Pentasa (registered trademark) was administered as granules obtained by pulversing Pentasa (registered trademark) tablet.

Results are shown in Fig.8 and Fig.9.

The compound [1] significantly reduced the damage score at a dose of 61.8 mg/kg (corresponding to 30 mg/kg as 5-ASA), and inhibited the increase of the wet weight of the colon significantly at a dose of 205.9 mg/kg (corresponding to 100 mg/kg as 5-ASA) (see Figs.8 and 9). On the other hand, Pentasa (registered trademark) did not show obvious effect on the damage score nor on the increase of the wet weight of the colon.

### Formulation example 1: Powdered drug (preparation for internal use)

In one preparation (700 mg)

| | |
|---|---|
| Compound [1] | 500 mg |
| Cornstarch | 127 mg |
| Crystalline cellulose | 35 mg |
| Polyvinyl alcohol | 35 mg |
| Magnesium stearate | 3 mg |

250 g of the compound [1], 63.5 g of cornstarch and 17.5 g of crystalline cellulose were fed to a fluid bed granulator/dryer, and 175 ml of 10% aqueous solution of polyvinyl alcohol was sprayed for granulation. Then, magnesium stearate (0.4% (w/w)) was added thereto, whereby a powdered drug containing 500 mg of this compound in 700 mg was obtained.

### Formulation example 2: Tablet (preparation for internal use)

In one preparation (600 mg)

| | |
|---|---|
| Compound [1] | 400 mg |
| Cornstarch | 153 mg |
| Crystalline cellulose | 42 mg |
| Magnesium stearate | 5 mg |

A powder mixture of 400 g of the compound [1], 153 g of cornstarch and 42 g of crystalline cellulose was compressed with a dry granulator and pulverized into granules. Then, magnesium stearate (0.8% (w/w)) was added thereto, and the mixture was formed into a tablet weighing 600 mg and having a diameter of 11 mm, whereby a tablet containing 400 mg of this compound was obtained.

### Formulation example 3: Capsule (preparation for internal use)

In one preparation (500 mg)

| | |
|---|---|
| Compound [1] | 250 mg |
| Anhydrous calcium hydrogen phosphate | 222.5 mg |
| Croscarmellose sodium | 25 mg |
| Magnesium stearate | 2.5 mg |

A powder mixture of 250 g of the compound [1], 222.5 g of anhydrous calcium hydrogen phosphate and 25 g of croscarmellose sodium was compressed with a dry granulator and pulverized into granules. Then, magnesium stearate (0.5% (w/w)) was added thereto, and 500 mg of the mixture was filled into a hard capsule No. 0, whereby a capsule containing 250 mg of this compound was obtained.

### Formulation example 4: Cylindrical granule (preparation for internal use)

In one preparation (1000 mg)

| | |
|---|---|
| Compound [1] | 750 mg |
| Cornstarch | 170 mg |
| Crystalline cellulose | 50 mg |
| Polyvinyl alcohol | 30 mg |

375 g of the compound [1], 85 g of cornstarch and 25 g of crystalline cellulose were fed to a kneader, and 125 ml of 12% aqueous solution of polyvinyl alcohol was added thereto and the mixture was kneaded. Then, the kneaded matter was extruded with a granule extrusion molding machine fitted with a screen having 0.7 mm diameter openings. The extruded matter was dried and sized, whereby a granule containing 750 mg of this compound in 1000 mg was obtained.

### Formulation example 5: Spherical coated granule (preparation for internal use)

In one preparation (1000 mg)

| | |
|---|---|
| Nonpareil | 200 mg |
| Compound [1] | 500 mg |
| Cornstarch | 170 mg |
| Low-substituted hydroxypropyl cellulose | 40 mg |
| Hydroxypropyl cellulose | 50 mg |
| Hydroxypropyl methyl cellulose | 30 mg |
| Propylene glycol | 6 mg |
| Titanium oxide | 4 mg |

200 g of nonpareil (24 to 32 mesh) was fed to a centrifugal fluidized granulation coating machine, and while spraying 8% aqueous solution of hydroxypropyl cellulose (50% ethanol), a powder mixture of 500 g of the compound [1], 170 g of cornstarch and 40 g of low-substituted hydroxypropyl cellulose was gradually added thereto for granulation, and the resulting granule was dried to obtain about 900 g of spherical granule base was obtained.

Then, 400 g of this spherical granule base was fed to a fluid bed granulator/dryer, and 250 ml of an aqueous solution containing 12.5 g of hydroxypropyl methyl cellulose, 2.5 g of propylene glycol and 1.7 g of titanium oxide was sprayed, whereby a coated granule containing 500 mg of this compound in 1000 mg was obtained.

### Industrial Applicability

The compound of the present invention has a characteristic feature that allows 5-ASA useful as a therapeutic agent for ulcerative colitis to be efficiently delivered to the large intestine as the site of action and does not allow 5-ASA to be transferred to the plasma. In other words, it can reduce systemic side effects, and also can increase the dose until the maximum therapeutic effect is obtained.

## Claims

1. 5-Amino-2-(β-D-galactopyranosyloxy)benzoic acid or 5-amino-2-(α-D-galactopyranosyloxy)benzoic acid or a pharmaceutically acceptable salt thereof.

2. A pharmaceutical composition comprising as an active ingredient 5-amino-2-(β-D-galactopyranosyloxy) benzoic acid or 5-amino-2-(α-D-galactopyranosyloxy) benzoic acid or a pharmaceutically acceptable salt thereof.

3. A therapeutic agent for ulcerative colitis comprising as an active ingredient 5-amino-2-(β-D-galactopyranosyloxy) benzoic acid, 5-amino-2-(α-D-galactopyranosyloxy) benzoic acid or 5-amino-2-(β-D-glucopyranosyloxy) benzoic acid or a pharmaceutically acceptable salt thereof.
